# EUROPÄISCHE PATENTSCHRIFT

(11) **EP 1 581 155 B1**
(45) Veröffentlichungstag und Bekanntmachung des Hinweises auf die Patenterteilung: **21.01.2009**
(21) Anmeldenummer: 03765065.2
(22) Anmeldetag: 21.07.2003
(51) Int. Cl.: A61F 7/12

(54) **VORRICHTUNG ZUR WÄRMEBEHANDLUNG DES MENSCHLICHEN VAGINAL- ODER KOLONBEREICHS**
DEVICE FOR THE THERMAL TREATMENT OF THE HUMAN VAGINAL OR COLON AREA
DISPOSITIF POUR LE TRAITEMENT PAR LA CHALEUR DE LA ZONE DU VAGIN OU DU COLON CHEZ L'ETRE HUMAIN

(30) Priorität: 23.07.2002 DE 20211045 U
(43) Veröffentlichungstag der Anmeldung: 05.10.2005
(73) Patentinhaber: Nordmann, Michael, 48268 Greven (DE)
(72) Erfinder: Nordmann, Michael, 48268 Greven (DE)
(86) Internationale Anmeldenummer: PCT/EP2003/007922
(87) Internationale Veröffentlichungsnummer: WO 2004/009004

(56) Entgegenhaltungen:
- EP-A- 0 715 837
- DE-U- 9 401 912
- DE-U- 9 420 260
- DE-U- 29 502 489
- GB-A- 1 360 999
- US-A- 3 170 465
- US-A- 3 939 842
- US-A- 5 094 238
- US-A- 5 417 721
- US-A- 5 993 480
- US-B1- 6 254 613

## Beschreibung

Die Erfindung betrifft eine Vorrichtung zur Wärmebehandlung des menschlichen Vaginal- oder Kolonbereichs, aufweisend einen länglichen Körper, der vorher erwärmt im benutzten Zustand ohne Zufuhr von Wärmeenergie wärmeabgebend wirkt.

Eine Vorrichtung der eingangs genannten Art ist aus DE 200 12 805.1 des Anmelders bekannt. Die Vorrichtung erfüllt ihre Aufgabe, jedoch hat sich gezeigt, dass in manchen Fällen dem Bedarf des Patienten gegenüber überdimensioniert und daher in Benutzung umständlich ist.

Es stellt sich demnach die Aufgabe, eine Vorrichtung der eingangs genannten Art dahingehend zu verbessern, dass diese praktisch in alle Körperhöhlen hineinpasst, wobei ihre Handhabung sehr einfach und diskret sein soll.

Diese Aufgabe wird bei der Vorrichtung gemäß dem Anspruch 1 gelöst. Nämlich durch eine Vorrichtung zur Wärmebehandlung des menschlichen Vaginal- oder Kolonbereichs, die aus einem länglichen, in die menschliche Körperhöhle gänzlich einführbaren und mit Hilfe eines Zugmittels (2) aus der Körperhöhle herausziehbaren, anatomisch angepassten Körper besteht, der im benutzten Zustand ohne Zufuhr von Wärmeenergie wärmeabgebend wirkt.

Der Körper ist hohl und mit einer flüssigen Mischung aus Gel und pulverisiertem Torf befüllt.

Der Körper kann ein metallischer Behälter sein.

Das Zugmittel kann ein Faden oder Bändchen sein, das vorzugsweise aus weichen, jedoch ausreichend reißfesten Textilfasern gefertigt ist. Das Zugmittel kann mit nur einem oder mit beiden seinen Enden am Körper befestigt sein. Vorzugsweise ist das Zugmittel ein mit dem Körper verbundenes Schwänzchen, das an seinem freien Ende eine die Handhabung erleichternde, beispielsweise kugelförmige Verdickung trägt.

Der Körper kann gänzlich oder nur an einem Teil seiner Lange zylindrisch bzw. walzenförmig sein. Im letzteren Fall kann dieser an einem weiteren Teil seiner Lange, der mit der sanft abgerundeten, anatomisch angeformten Spitze endet, etwa ellipsoidal bis kugelförmig ausgebildet sein.

Bei einer anderen Ausführungsform kann der Körper eine mittig angeordnete Verdickung aufweisen, die beidseitig jeweils in eine sanft abgerundete Spitze übergeht.

Der Behälter ist nicht verformbar, falls dieser aus Metall oder Hartkunststoff gefertigt ist, wobei sich das Adjektiv "verformbar" auf die Formgebung, nicht aber auf temperaturbedingte Maßveränderungen bezieht. So kann der metallische Behälter aus Stahl, Aluminium, Titan oder deren Legierungen hergestellt sein.

Es gibt auch ein Ensemble, welches aus wenigstens einer bereits beschriebenen Vorrichtung und einem vorzugsweise elektrisch beheizbaren Erhitzer besteht, in den die Vorrichtung platziert und auf gewünschte Temperatur erwärmt werden kann. Zu diesem Zweck ist der Erhitzer mit einem Thermostat ausgestattet. Ein solcher Erhitzer ist einem bekannten Flaschen- und Babykostwärmer ähnlich.

Der Erhitzer besitzt einen Behälter zur Aufnahme der Vorrichtung und einen abnehmbaren oder verschwenkbaren Verschlußdeckel, so dass die Vorrichtung auch geschlossen in dem Erhitzer tagsüber steril gehalten werden kann.

Es sei darauf hingewiesen werden, dass sich die Vorrichtung gemäß der Erfindung auch zur thermotheraupetischen Behandlung von Hämmorhoiden und Prostata, beispielsweise zur Behandlung von Prostata-Hyperplasie (BPH), bei der eine Temperatur bis 48°C verwendet wird, eignet.

Ausführungsbeispiele der Erfindung und andere Beispiele, auf die sich die Erfindung nicht Bezieht, sind in der nachfolgenden Beschreibung anhand der Zeichnung dargestellt. Die Figuren zeigen im einzelnen:
- Fig. 1: eine Vorrichtung zur Wärmebehandlung in einer perspektivischen Sicht;
- Figuren 2a bis 2c: die Vorrichtung gemäß Fig. 1 mit einer hohlen Verschlußkappe, in einem längsaxialen Schnitt;
- Fig. 3: eine walzenförmige Vorrichtung im Teilschnitt;
- Fig. 4: eine zweitelige Vorrichtung mit einer mittig angeordneten Verdickung;
- Fig. 5: Draufsicht auf eine Verschlußkappe mit Mulden;
- Fig. 6: eine Vorrichtung in massiver Ausführung, in Teilschnitt;
- Fig. 7: eine Vorrichtung mit einem hohlen Körper;
- Fig. 8: ein Ensemble in einem Halbschnitt.

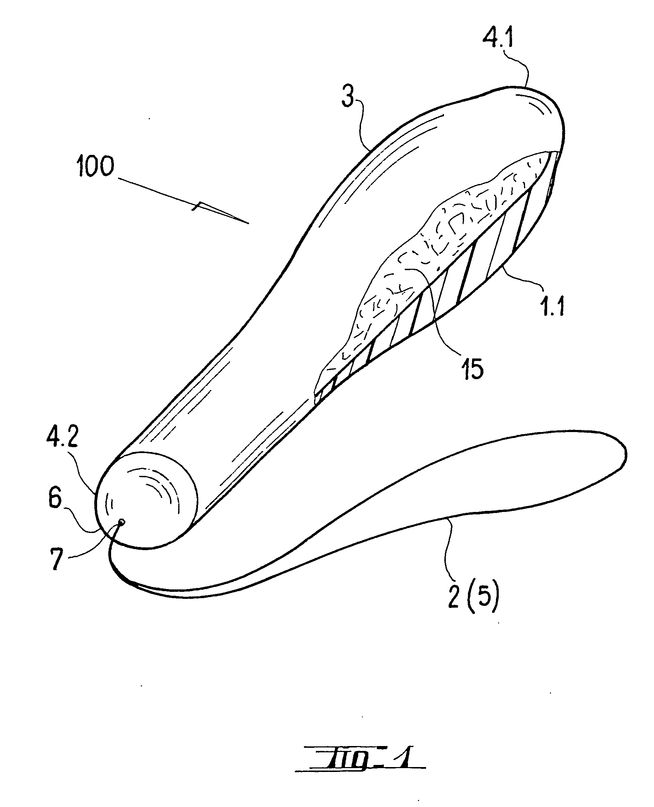

In Fig. 1 ist eine Vorrichtung 100 dargestellt, bestehend aus einem zweiteiligen, hohlen, länglichen Körper 1.1 mit Füllung 15 und aus einem Zugmittel 2. Der Körper 1.1 weist an einem Teil seiner Länge eine ellipsoidale Verdickung 3 auf, die in eine sanft abgerundete, anatomisch angepasste Spitze 4.1 übergeht. Am übrigen Teil seiner Länge ist der Körper 1.1 walzenförmig und endet mit einer Verschlußkappe 6, die wiederum eine zweite sanft abgerundete Spitze 4.2 bildet.

Das Zugmittel 2 ist ein reißfester Faden aus Baumwolle, welcher durch eine an der Verschlußkappe 6 eingearbeitete Bohrung 7 durchgezogen und mit einem sich im Inneren der Verschlußkappe befindenden Anschlagelement in Form eines Knotens oder einer Kugel 16 verbunden ist.

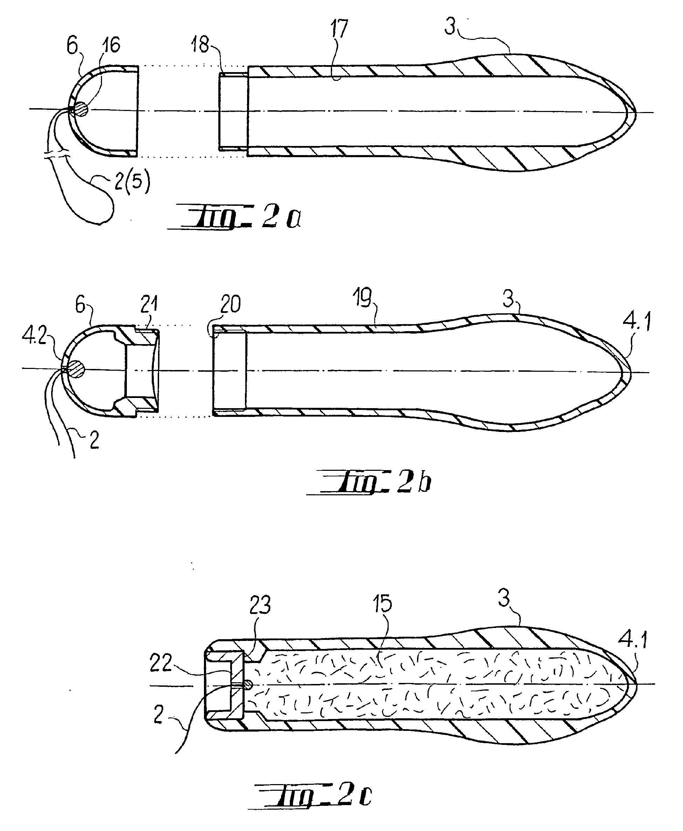

Die Vorrichtung 100 entspricht im wesentlichen den in Figuren 2a bis 2c gezeigten axialen Längsschnitten. Der längliche Körper gemäß Fig. 2a weist eine zylindrische, axiale, innere Kammer 17 und einen der Spitze 4.1 gegenüberliegenden, axialen Vorsprung 18 zum Aufsetzen der kalottenförmigen Verschlußkappe 6 auf. Die Kammer 17 wird mit einem dickflüssigen, wärmespeichernden Medium gefüllt (Füllung 15) und danach die Verschlußkappe 6 am Vorsprung aufgesetzt. Das Zugmittel 2 bildet eine geschlossene

Schlaufe 5. Die Verschlußkappe 6 wird mit dem Körper 1.1 verklebt, so dass die Füllung nicht mehr ausgetauscht werden muss.

Als dickflüssiges Medium eignen sich beispielsweise Gele auf Methylcellulose-Basis oder eine Mischung von ausgetrocknetem und pulverisiertem Moor mit Wasser.

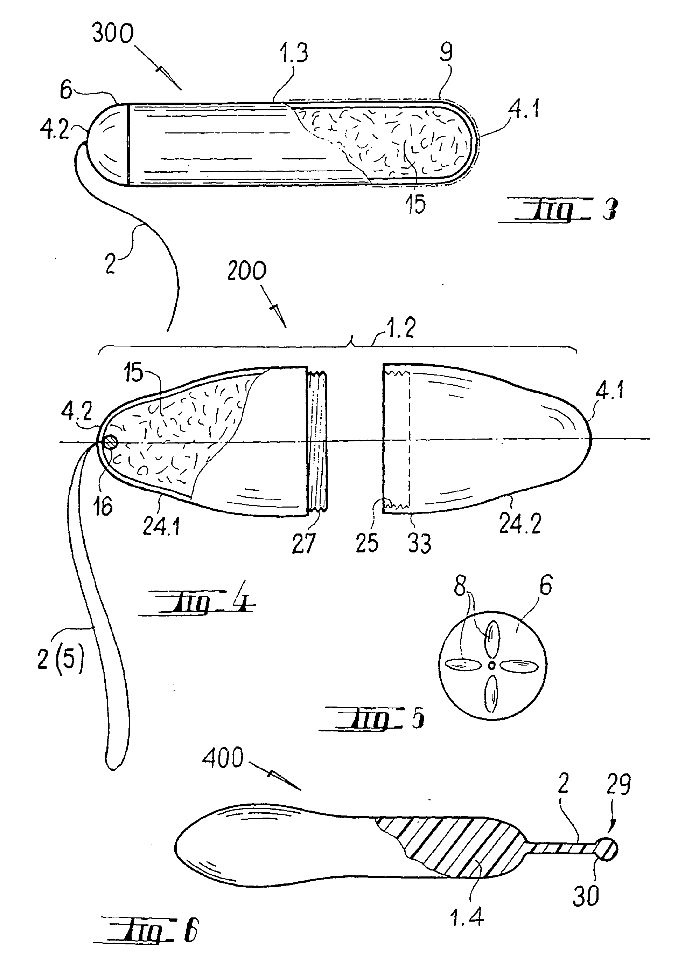

Der längliche Körper mit Verdickung 3 gemäß Fig. 2b weist eine Wandung 19 von gleichmäßiger Dicke und ein Innengewinde 20 auf, das etwa 10 mm ins Innere des Körpers reicht. Die Verschlußkappe 6 endet mit einem an das Innengewinde angepassten Gewindestutzen 21. Zur Verbesserung der Handhabung beim Ein- oder Abschrauben der Verschlußkappe ist diese mit drei radial angeordneten Mulden 8 versehen (vgl. Fig. 5).

Eine etwas abweichende Ausführungsform der Vorrichtung ist in Fig. 2c dargestellt. Eine etwa becherförmige Verschlußkappe 6 ist mit ihrem Boden 22 in einen am länglichen Körper angeordneten Sitz 23 hineingepresst. Insgesamt ist der der Spitze 4.1 abgewandte Endbereich der Vorrichtung ebenfalls sanft abgerundet.

In Fig. 3 ist eine Vorrichtung 300 mit einem zylindrischen Körper 1.3, d.h. ohne Verdickung gezeigt. Der aus Aluminium hergestellte Körper 1.3 weist ebenso zwei sanft abgerundete Spitzen 4.1, 4.2 und eine kalottenförmige Verschlußkappe auf. Der Körper 1.3 ist von einer Umhüllung 9 aus einer schleimhautverträglichen, sterilisierbaren Kunststoff-Folie umgeben.

Die Fig. 4 zeigt eine Vorrichtung 200, aufweisend einen zweiteligen Körper 1.2 mit Füllung 15 und ein Zugmittel 2. Der Körper 1.2 besteht aus zwei formgleichen Bechern 24.1, 24.2, die nach dem Zusammenfügen eine mittige Verdickung 33 bilden. Im vorliegenden Fall weist einer Becher 24.1 einen Gewindestutzen 27 und der zweite Becher

24.2 ein Innengewinde 25 auf, so dass die Becher miteinander verschraubt werden können. Anstelle der Verschraubung kann eine Rastverbindung oder Verklebung, wie bei den bekannten Tischtennis-Bällen der Fall ist, vorgenommen werden. Als Füllung 15 wird ein offenporiger Weichschaum verwendet, der mit einer flüssigen wärmespeichernden Substanz getränkt ist.

Sowohl der Körper 1.1; 1.2 als auch ein massiver Körper 1.4 einer in der Fig. 6 dargestellten, weiteren Vorrichtung 400 ist aus einem thermoplastischen Elastomer-Compound "Evoprene G" (Handelsname der Firma Evode Plastics) hergestellt. Das Material zeichnet sich durch gute mechanische Eigenschaften, leichte Verarbeitkeit, hohe Wärmeformbeständigkeit und durch einen niedrigen Druckverformungsrest aus. Der aus diesem Kunststoff hergestellte Körper ist desinfizierbar und kann im Heißwasser gereinigt werden.

An den massiven Körper 1.4 ist ein Zugmittel 2 in Form eines in eine kugelförmige Verdickung 30 auslaufenden Schwänzchens angeschlossen. Wie die Fig. 6 zeigt, ist das Schwänzchen einstückig mit dem übrigen Körper 1.4 ausgeführt. Mit der Verdickung 30 lässt sich die Vorrichtung 400 mühelos aus der Körperhöhle herausziehen.

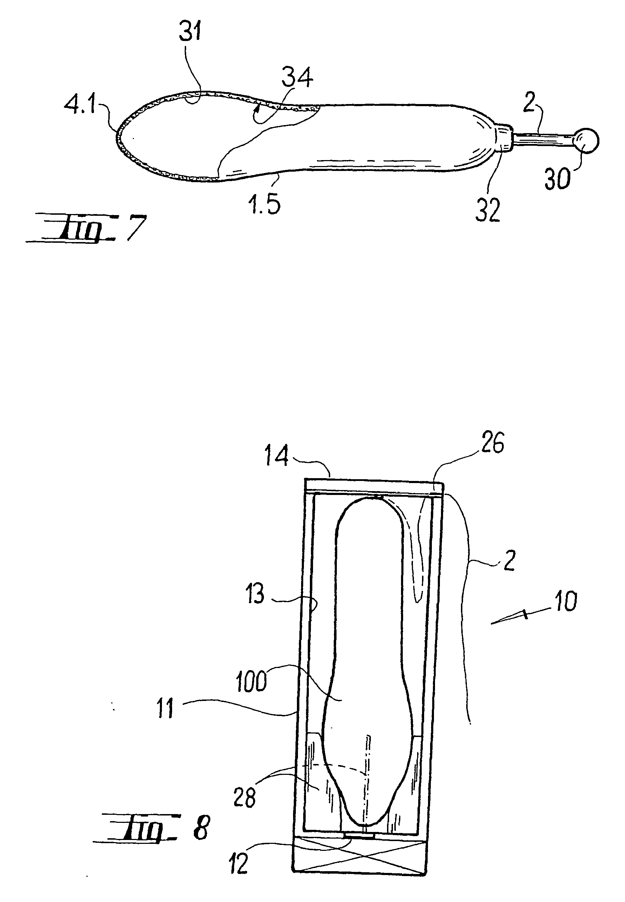

Schließlich zeigt die Fig. 7 einen hohlen Körper 1.5, dessen abgerundetes, der Spitze 4.1 abgewandtes Ende über eine Abstufung 32 in das Schwänzchen (Zugmittel 2) übergeht. Das Schwänzchen trägt ebenso eine kugelförmige Verdickung 30. Vorzugsweise ist die Abstufung 32 etwas abgerundet. Die Abstufung 32 kann auch bei der Ausführungsform gemäß der Fig. 6 Verwendung finden.

Der Körper 1.5 ist an seiner Innenfläche 34 mit einer thermoplastischen Masse beschichtet, in der Mikrokapseln 31 dispergiert sind. Die Mikrokapseln 31 von einem Durchmesser von 8 µm bis 200 µm enthalten eine wärmespeichernde Öl-Wachs-Komposition.

Die Vorrichtung 100; 200; 300; 400 kann in einem Wasserbad oder in einem Mikrowellenoffen erwärmt werden.

Bei der Verwendung eines massiven Körpers 1.4 wird empfohlen, die Vorrichtung ins kochendes Wasser einzutauchen, nach einigen Minuten herauszunehmen und bis 3 Minuten abkühlen lassen. Die Temperatur eines etwa 13 cm langen massiven Körpers beträgt 57°C.

Bevorzugt wird jedoch ein handlicher Erhitzer 11, der einen elektrisch beheizbaren, zylindrischen Behälter 13 mit einem Verschlußdeckel 14 besitzt (vgl. Fig. 8). Im Behälter wird die Vorrichtung 100; 200; 300; 400 so untergebracht, dass das Zugmittel 2 durch eine am Rand des Deckels, bzw. Behälters eingearbeitete, kleine Auskehlung 26 nach außen geführt wird, damit die Vorrichtung einfacher aus dem Behälter entnommen werden kann. Auf die Auskehlung kann verzichtet werden.

Unterhalb des Behälters ist ein nicht dargestelltes Heizelement und ein Thermostat 12 angeordnet. Optional können in den Behälter Stege 28 eingelegt werden, an denen sich die Vorrichtung abstützen kann. Der Erhitzer 11 und die Vorrichtung 100; 200; 300; 400 bilden ein Ensemble 10, das unauffällig im Bad oder im Nachtschränkchen neben dem Bett des Patienten aufbewahrt werden kann. Zur Ausstattung des Ensemble 10 können auch marktübliche Sterilisiertabletten gehören.

### Bezugszeichenliste:

- 1.1; ....1.5: Körper
- 2: Zugmittel
- 3: Verdickung
- 4.1, 4.2: Spitze
- 5: Schlaufe
- 6: Verschlußkappe
- 7: Bohrung
- 8: Mulde
- 9: Umhüllung
- 10: Ensemble
- 11: Erhitzer
- 12: Thermostat
- 13: Behälter
- 14: Verschlußdeckel
- 15: Füllung
- 16: Kugel
- 17: Öffnung
- 18: Vorsprung
- 19: Wandung
- 20: Innengewinde
- 21: Gewindestutzen
- 22: Boden
- 23: Sitz
- 24.1; 24.2: Becher
- 25: Innengewinde
- 26: Auskehlung
- 27: Gewindestutzen
- 28: Steg
- 29: Ende
- 30: Verdickung (Kugel)
- 31: Mikrokapsel
- 32: Abstufung
- 33: Verdickung
- 34: Innenfläche
- 100; 200; 300; 400: Vorrichtung

## Patentansprüche

1. Vorrichtung (100; 200) zur Wärmebehandlung des menschlichen Vaginal- oder Kolonbereichs, aufweisend einen länglichen, in die menschliche Körperhöhle gänzlich einführbaren und mit Hilfe eines Zugmittels (2) aus der Körperhöhle herausziehbaren, anatomisch angepassten Körper (1.1; 1.2; 1.3; 1.5), der hohl ist und im benutzten Zustand ohne Zufuhr von Wärmeenergie wärmeabgebend wirt,
**dadurch gekennzeichnet, dass**
der Körper mit einer flüssigen Mischung aus Gel und pulverisiertem Torf befüllt ist.

2. Vorrichtung nach Anspruch 1, **dadurch gekennzeichnet, dass** der Körper (1.3) ein metallischer Behälter ist, der mit einer Umhüllung (9) aus einer schleimhautverträglichen, sterilisierbaren Kunststoff-Folie umgeben ist.

3. Vorrichtung nach Anspruch 1, **dadurch gekennzeichnet, dass** mittig am Körper eine Verdickung (33) angeordnet ist.

4. Vorrichtung nach Anspruch 1 und 3, **dadurch gekennzeichnet, dass** der Körper (1.2) aus zwei formgleichen Bechern (24.1, 24.2) besteht.

## Claims

1. Device (100; 200) for the thermal treatment of the human vaginal or colonic region, comprising a hollow, elongated, anatomically adapted element which can be fully inserted into and retracted from the body cavity with the assistance of a drawing means and, when in the operative state, has a warmth-releasing effect without the supply of additional thermal energy, and
is distinguished by the fact that its body is filled with a fluid mixture of gel and powdered peat.

2. Device as per Claim 1., distinguished by the fact that the body (1.3) is a metallic vessel is wrapped in mucous-friendly encasement of sterilizable plastic film 9.

3. Device as per Claim 1., distinguished by the fact the middle of the body is bulged (33).

4. Device as per Claims 1. and 3., distinguished by the fact that the body (1.2) consists of two form-identical cups (24.1, 24.2).

## Revendications

1. Dispositif (100; 200) pour le traitment thermique des zones humaines vaginales et cloniques, constitué d'un corps creux oblong (1.1; 1.2; 1.3; 1.5), à conformité anatomique, introduis complètement dans l'orifice humaine et retiré avec l'assistance d'un moyen d'extraction, qui, dans son mode d'emploi, dégage une chaleur sans approvisionnement supplémentaire d'énergie thermique et
qui se distingue par un corps remplis d'un mélange fluide de gel et de tourbe pulverisée.

2. Dispositif de la revendication1., qui se distingue par le fait que le corps (1.3) est un récipient métallique couvert d'une couche (9) de film plastique conviviale à la muqueuse et sterilisable.

3. Dispositif de la revendication 1., qui se distingue par le fait que le corps démontre un renflement central (33).

4. Dispositiv de la revendication 1. et 3., qui se distingue par le fait que le corps (1.2) consiste de deux gobelets de forme identique (24.1, 24.2).
